(19) Europäisches Patentamt
European Patent Office
Office européen des brevets

(11) Numéro de publication: **0 360 642 A2**

(12) # DEMANDE DE BREVET EUROPEEN

(21) Numéro de dépôt: **89402332.4**

(51) Int. Cl.[5]: **A 61 K 31/435**

(22) Date de dépôt: **24.08.89**

(30) Priorité: **24.08.88 FR 8811157**

(43) Date de publication de la demande: **28.03.90 Bulletin 90/13**

(84) Etats contractants désignés: **AT BE CH DE ES FR GB GR IT LI LU NL SE**

(71) Demandeur: **ADIR ET COMPAGNIE**
**22, rue Garnier**
**F-92201 Neuilly sur Seine (FR)**

(72) Inventeur: **Teisseire, Bernard**
**28, rue de Gourmant**
**F-75019 Paris (FR)**

Revendications pour les Etats contractants suivants: GR + ES.
Le titre de l'invention a été modifié (Directives relatives à l'examen pratiqué à l'OEB, A-III, 7.3)

(54) **Dérivés de l'acide aza-2 bicyclooctane(2,2,2)carboxylique-3 pour le traitement de maladies vasculaires.**

(57) Utilisation de dérivés de l'acide aza-2 bicyclooctane [2.2.2] carboxylique-3 et leurs sels pour l'obtention de médicaments destinés au traitement de l'artérite, et plus particulièrement de l'artérite des membres inférieurs, des troubles de la circulation cérébrale et plus particulièrement des troubles de la sénescence cérébrale et la migraine, troubles de la vision tels que troubles rétiniens d'origine vasculaire, rétinopathies diabétiques, atrophies choriorétiniennes, dégénérescence choriorétinienne.

EP 0 360 642 A2

**Description**

## UTILISATION DE DERIVES DE L'ACIDE AZA - 2 BICYCLOOCTANE [2.2.2] CARBOXYLIQUE - 3 POUR L'OBTENTION DE MEDICAMENTS DESTINES AU TRAITEMENT DES MALADIES CONSECUTIVES A L'ALTERATION DE LA PAROI VASCULAIRE AINSI QU'AUX TROUBLES DE LA MICROCIRCULATION

La présente invention concerne l'utilisation de dérivés de l'acide aza - 2 bicyclooctane [2.2.2] carboxylique - 3 pour l'obtention de médicaments destinés au traitement de maladies vasculaires.

Plus spécifiquement, l'invention concerne l'utilisation, pour l'obtention de médicaments destinés au traitement de maladies résultant d'altérations de la paroi vasculaire ou de troubles de la microcirculation, de dérivés de formule générale (I) :

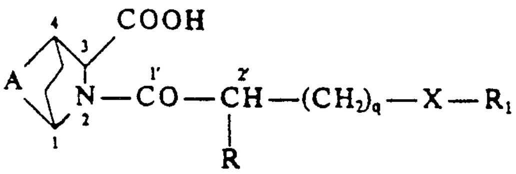

(I)

dans laquelle :
A représente un radical vinylène ou diméthylène,
q est 0 ou 1,
R représente un radical alkyle inférieur pouvant porter un groupe amino,
X représente - S - et $R_1$ représente H, ou bien X représente - NH - et,
$R_1$ représente un atome d'hydrogène ou un radical de formule :

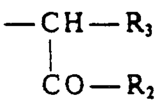

$R_2$ représente un hydroxyle ou un groupement alcoxy inférieur,
$R_3$ représente un atome d'hydrogène, un radical alkyle linéaire ou ramifié, cycloalkyle ou phénylalkyle, ayant au total un maximum de 8 atomes de carbone, ou un radical de formule :

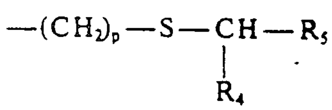

dans lequel :
$R_4$ est H, un radical alkyle inférieur ou cycloalkyle ($C_3$ à $C_6$),
$R_5$ est H, un radical cycloalkyle ($C_3$ à $C_6$) ou alcoxy (inférieur) - carbonyle, et
p est 1 ou 2,
étant entendu, que radical alkyle ou alcoxy inférieur représente des groupes ayant de 1 à 4 atomes de carbone, ainsi que leurs sels d'addition obtenus avec une base minérale ou organique, thérapeutiquement compatible ou avec un acide minéral ou organique, thérapeutiquement compatible lorsque X représente NH.

Comme base pouvant salifier les composés de formule (I), on pourra utiliser des hydroxydes de sodium, potassium, calcium ou d'aluminium, des carbonates de métaux alcalins ou alcalino-terreux ou des bases organiques comme la triéthylamine, la benzylamine, la diéthanolamine, la tert.butylamine, la dicyclohexylamine, l'arginine, etc...

Parmi les acides que l'on peut ajouter aux composés de formule (I) pour obtenir un sel d'addition on peut citer, à titre non limitatif les acides chlorhydrique, sulfurique, phosphorique, tartrique, malique, maléique, fumarique, oxalique, méthane-sulfonique, éthane-sulfonique, camphorique, citrique, etc..

Les composés de formule (I) comportent au moins deux atomes de carbone asymétrique. L'utilisation selon l'invention peut concerner les différents isomères des dérivés de formule (I) isolés ou même en mélange. Toutefois, l'utilisation selon l'invention concerne préférentiellement les dérivés de formule (I) dans lesquels les atomes de carbone asymétrique ont la configuration (S).

Les composés de formule (I) préférentiellement utilisables selon l'invention sont ceux pour lesquels :
A représente un groupement diméthylène,
q est 0,
R représente un groupement méthyle ou amino - 4 butyle,
X représente NH, et
$R_1$ représente un radical de formule :

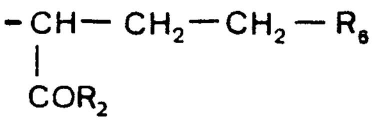

$R_2$ possédant la définition indiquée précédemment,
$R_6$ représentant un groupement méthyle, éthyle ou phényle ainsi que leurs sels d'addition.

Parmi ceux-ci, celui pour lesquels $R_2$ signifie éthoxy et $R_6$ est un radical phényle, et R représente un groupement méthyle est préféré. Il s'agit du (S,S,S) [(phényl - 3 éthoxycarbonyl - 1 propyl) amino - 2 propionyl] - 2 aza - 2 carboxy - 3 bicyclo [2.2.2] octane.

Les composés de formule (I) sont décrits dans le brevet européen 0 051 020 ainsi que dans la demande de brevet européen 0 105 102 comme étant utilisables dans le traitement de l'hypertension artérielle à des posologies comprises entre 1 et 200 mg.

La demanderesse a présentement découvert que les produits de formule (I) possédaient d'intéressantes propriétés applicables à l'obtention de médicaments destinés au traitement des maladies dues à une altération de la paroi vasculaire ainsi qu'au traitement des maladies consécutives à des troubles de la microcirculation et ceci à des doses n'entraînant pas d'activité antihypertensive.

Parmi les affections susceptibles d'être traitées par les médicaments obtenus selon l'invention, il convient de citer l'artérite, l'artérite des membres inférieurs, ainsi que les troubles de la circulation cérébrale, et particulièrement les troubles de la sénescence cérébrale et la migraine, troubles de la vision tels que troubles rétiniens d'origine vasculaire, rétinopathies diabétiques, atrophies choriorétiniennes, dégénérescence choriorétinienne.

Les médicaments, destinés au traitement des maladies consécutives à l'altération de la paroi vasculaire ainsi qu'aux troubles de la microcirculation, obtenus en utilisant selon l'invention les composés de formule (I) ou leurs sels pharmaceutiquement acceptables, seront présentés sous des formes pharmaceutiques convenant pour l'administration par voie orale, parentérale et notamment intraveineuse, per ou transcutanée, nasale, rectale, perlinguale, oculaire ou respiratoire et notamment les préparations injectables, les aérosols, les gouttes oculaires ou nasales, les comprimés, les comprimés sublinguaux, les glossettes, les gélules, les capsules, les tablettes, les suppositoires, les crèmes, pommades, gels dermiques etc...

La posologie varie selon l'âge et le poids du patient, la voie d'administration, la nature de l'indication thérapeutique et des traitements associés et s'échelonne de 0,001 mg à 10 mg par prise ou par application.

Les exemples suivants illustrent l'invention et ne la limitent en aucune façon.

**EXEMPLE 1 : ACTIVITE DES PRODUITS DE FORMULE (I) A DOSES NON HYPOTENSIVES SUR L'ENZYME DE CONVERSION PLASMATIQUE**

L'utilisation d'un dérivé de formule (I) - ayant des propriétés inhibitrices de l'enzyme de conversion - dans l'obtention de compositions pharmaceutiques destinées au traitement des maladies consécutives à l'altération de la paroi vasculaire ainsi qu'aux troubles de la microcirculation suppose que ce produit soit actif sur l'enzyme de conversion à doses non hypotensives.

Une étude a donc été réalisée sur des rats Sprague Dawley de 80 à 100 grammes. Ce groupe d'animaux a reçu un produit de formule (I) dans l'eau de boisson, à raison de 0,1 $mg.kg^{-1}$ ; un autre groupe d'animaux n'a pas été traité. Après 7 jours de traitement, les rats sont anesthésiés par 50 $mg.kg^{-1}$ de pentobarbital sodique. Un cathéter est introduit dans la carotide pour le recueil de deux échantillons, l'un sur tube sec pour le dosage de l'activité de l'enzyme de conversion, l'autre sur EDTA pour le dosage de l'angiotensine I. Après recueil, les prélèvements sont immédiatement centrifugés à 4° C et 3000 RPM durant 10 minutes. Les surnageants sont prélevés, congelés à -80° C en attente du dosage. Il résulte que 7 jours d'administration per os du produit de formule (I) à 0,1 $mg.kg^{-1}$/jour conduisent à une inhibition de 52% de l'enzyme circulatoire. Ce résultat prouve que les produits de formule (I) agissent sur l'enzyme de conversion à doses non hypotensives.

**EXEMPLE 2 : DEMONSTRATION DE L'EXISTENCE D'UN SYSTEME RENINE ANGIOTENSINE A L'INTERIEUR DE LA PAROI VASCULAIRE SENSIBLE AUX PRODUITS DE FORMULE (I)**

L'expérience à été réalisée sur les artères fémorale et saphène interne et la veine saphène de porcins. Des mini porcs (Charles-River) ont été anesthésiés par une injection intramusculaire de 1,5 $mg.kg^{-1}$ de Stressnil et intrapéritonéale de pentobarbital sodique (40 $mg.kg^{-1}$) et vidés de leur sang.

Après avoir été recueillis, les vaisseaux sanguins ont été débarrassés du tissu conjonctif adhérent et coupés en segments de 4 mm de long environ, conservés en milieu bicarbonaté de Krebs-Ringer modifié.

La tension isométrique a été mesurée par un indicateur tensiomètre (Gould $UC_2$) et relié à un enregistreur (Gould). Les segments vasculaires ont été amenés au point optimal de leur rapport longueur/tension par des stimulations répétées au KCL40m M.

Les segments de vaisseaux ont été laissés au repos aux fins d'équilibre de la préparation 45 minutes avant le début de chaque expérience. L'endothélium vasculaire a été éliminé de certains échantillons par un léger frottement de la surface de l'intima à l'aide d'un petit forceps.

On a montré que l'angiotensinogène, l'angiotensine I ($1.10^{-10}$, 3. $10^{-7}$ M) et l'angiotensine II ($10^{-10}$, $10^{-7}$ M) induisent des contractions des artères fémorales. Ces contractions sont dépendantes de la présence de l'endothélium pour l'angiotensinogène et l'angiotensine I ; un système complet Rénine-Angiotensine existe

donc dans la paroi vasculaire.

On a également montré, que les produits de formule (I) agissent essentiellement sur les enzymes de conversion présents dans l'endothélium vasculaire, ce qui prouve que les produits de formule (I) sont susceptibles d'être utilisés dans l'obtention de médicaments destinés au traitement des troubles affectant cette paroi vasculaire.

**EXEMPLE 3 : ACTIVITE DES PRODUITS DE FORMULE (I) SUR LA MICROCIRCULATION ISCHEMIQUE**

L'étude expérimentale a été réalisée sur les muscles crémasters de rats mâles (Sprague - Dawley) après ligature de l'artère iliaque commune.

Les muscles ont été placés dans une chambre transparente, perfusés par une solution de tampon bicarbonate équilibrée par un mélange gazeux $CO_2/N_2$ 5/95 %. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesures simultanément :

- sur le crémaster perfusé normalement,
- sur le crémaster sous ligature, c'est à dire le crémaster ischémié 2, 7, 14 et 21 jours après la ligature.

Deux groupes d'animaux ont été étudiés :

- un groupe témoin sans traitement,
- un groupe traité per os par un produit de formule (I), à raison de 0,1 $mg.kg^{-1}$ par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de formule (I) améliore la microcirculation et l'irrigation sanguine des territoires ischémiés.

**EXEMPLE 4 : ACTIVITE DES COMPOSES DE FORMULE (I) SUR LES MOUVEMENTS TRANSMEMBRANAIRES DES MACROMOLECULES AU NIVEAU DES MICROVAISSEAUX DU MUSCLE ISCHEMIE**

On a étudié les effets in vivo des produits de formule (I) sur les mouvements transvasculaires des macromolécules induits par l'ischémie.

La préparation concernait le muscle crémaster de rat placé dans une chambre transparente et perfusé par une solution de tampon bicarbonate équilibrée par un mélange gazeux $CO_2/N_2$ 5%/95% de façon à obtenir un pH de 7,40.

On a injecté de la sérum albumine bovine (fraction IV) marquée par l'isothiocyanate de fluorescéine (FITC-BSA) comme traceur macromoléculaire (15 $mg.kg^{-1}$). L'ischémie a été réalisée pendant une heure par un clamp placé sur l'artère principale du muscle crémaster.

La clearance de la FITC-BSA a été calculée par le rapport de l'extravasation par unité de poids tissulaire sur la concentration plasmatique de FITC-BSA, ce résultat étant exprimé en pourcentage de la valeur moyenne obtenue pendant une période préalable de contrôle de 20 minutes.

Les animaux traités durant 3 jours par 1 $mg.kg^{-1}$ par jour per os d'un produit de formule (I), montrent une extravasation macromoléculaire sous bradykinine plus élevée que celle constatée chez les animaux témoins. Ces résultats démontrent l'effet inhibiteur d'un produit de formule (I) sur l'enzyme de conversion de l'angiotensine présent dans le système microcirculatoire musculaire.

Un traitement par un composé de formule (I) réduit de façon significative l'augmentation de la perméabilité microvasculaire observée in vivo consécutivement à une heure d'ischémie totale.

**EXEMPLE 5 : ACTIVITE DES COMPOSES DE FORMULE (I) SUR LES PROPRIETES ELASTIQUES DE L'AORTE DE RAT**

L'altération des propriétés élastiques d'aorte de rat a été étudiée dans trois groupes de rats Goldblatt.

On a traité des rats hypertendus durant cinq semaines avec un produit de formule (I), à raison de 0,1 $mg.kg^{-1}$ et 0,01 $mg.kg^{-1}$; leurs aortes ont été étudiées après cinq semaines et comparées aux aortes de rats n'ayant reçu aucun traitement.

Des fragments aortiques ont été placés en suspension dans des chambres organiques remplies d'une solution de bicarbonate de Krebs Ringer modifié. Ces fragments ont été traités par la papavérine ($10^{-4}$ M) et le nitroprussiate de sodium ($10^{-4}$ M) pour éliminer toute interférence dûe à la tension active développée par le muscle lisse. Des fragments aortiques de rats hypertendus ont développé une tension supérieure à celle des fragments aortiques de rats normotendus par un étirement identique.

Le traitement de rats opérés à blanc et normotendus par 0,1 $mg.kg^{-1}$ par jour du produit de formule (I), n'a pas entraîné de différences significatives avec les témoins, eux aussi opérés a blanc. Les fragments aortiques de rats hypertendus traités par 0.01 $mg.kg^{-1}$ par jour de produit de formule (I), ont développé une tension

inférieure à celle des animaux non traités.

Les résultats d'un tel test confirment les possibilités d'utilisation de médicaments obtenus à partir de composés de formule (I) dans les troubles de la paroi vasculaire.

**EXEMPLE 6 : EFFETS D'UNE ADMINISTRATION CHRONIQUE DE DOSES NON HYPOTENSIVES DE COMPOSES DE FORMULE (I) CHEZ LE RAT HYPERTENDU**

Cette étude réalisée chez un grand nombre de rats montre tout d'abord que l'apparition d'une hypertension consécutive à l'installation d'un clip sur l'artère rénale entraîne une augmentation parallèle et significative du poids de l'aorte (rat Goldblatt) (+21 %).

Les composés de formule (I) administrés à la dose de 0,1 $mg.kg^{-1}$ par jour, durant 5 semaines chez des rats opérés à blanc, ne modifient pas la pression artérielle, mais entraînent une diminution significative du poids de l'aorte (-14%) des rats traités en comparaison aux rats traités par placebo.

Ce test montre le rôle important que jouent les dérivés de formule (I) administrés chroniquement sur les propriétés de la paroi vasculaire.

**EXEMPLE 7 : COMPOSITION PHARMACEUTIQUE**

Formule de préparation pour 1000 comprimés dosés à 0,3 mg de principe actif.

| | |
|---|---|
| (S) [(S) ethoxy carbonyl - 1 phényl - 3 propylamino) - 2 oxo - 1 propyl] - 2 carboxy - 3 (S) aza - 2 bicyclo [2.2.2] octane . . . . . . . . . | 300 mg |
| Hydroxy propyl cellulose . . . . . . . . . . . . . . . . . . . . . | 2 g |
| Amidon de blé . . . . . . . . . . . . . . . . . . . . . . . . | 10 g |
| Lactose . . . . . . . . . . . . . . . . . . . . . . . . . . . | 100 g |
| Stéarate de magnésium . . . . . . . . . . . . . . . . . . . . | 3 g |
| Talc . . . . . . . . . . . . . . . . . . . . . . . . . . . . . | 3 g |

**Revendications**

1/ Utilisation, pour l'obtention de médicaments destinés au traitement de l'artérite et plus particulierement de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) :

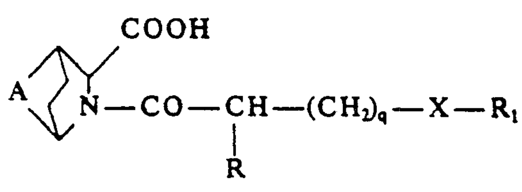

(I)

dans laquelle :

A représente un radical vinylène ou diméthylène,

q est 0 ou 1,

R représente un radical alkyle inférieur pouvant porter un groupe amino,

X représente - S - et $R_1$ représente H, ou bien X représente - NH - et,

$R_1$ représente un atome d'hydrogène ou un radical de formule :

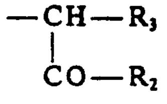

$R_2$ représente un hydroxyle ou un groupement alcoxy inférieur,
$R_3$ représente un atome d'hydrogène, un radical alkyle linéaire ou ramifié, cycloalkyle ou phénylalkyle, ayant au total un maximum de 8 atomes de carbone, ou un radical de formule :

$$-(CH_2)_p-S-CH-R_5$$
$$|$$
$$R_4$$

dans lequel :
$R_4$ est H, un radical alkyle inférieur ou cycloalkyle ($C_3$ à $C_6$),
$R_5$ est H, un radical cycloalkyle ($C_3$ à $C_6$) ou alcoxy (inférieur) - carbonyle, et
p est 1 ou 2,
étant entendu, que radical alkyle ou alcoxy inférieur représente des groupes ayant de 1 à 4 atomes de carbone,
ainsi que leurs sels d'addition obtenus avec une base minérale ou organique thérapeutiquement compatible ou avec un acide minéral ou organique thérapeutiquement compatible lorsque X représente NH.

2/ Utilisation, selon la revendication 1, pour l'obtention de médicaments destinés au traitement de l'artérite, de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) dans lequel les carbone dits asymétriques ont la configuration S, ainsi que leurs sels d'addition obtenus avec une base minérale ou organique thérapeutiquement compatible ou avec un acide minéral ou organique thérapeutiquement compatible lorsque X représente NH.

3/ Utilisation, selon les revendications 1 et 2, pour l'obtention de médicaments destinés au traitement de l'artérite et plus particulièrement de l'artérite des membres inférieurs, des troubles de la circulation cérébrale, et particulièrement des troubles de la sénescence cérébrale et la migraine, et des troubles de la vision tels que troubles rétiniens d'origine vasculaire, rétinopathies diabétiques, atrophies choriorétiniennes, dégénérescence choriorétinienne, de composés de formule (I) selon la revendication 1 et 2 dans lesquels :
A représente un radical diméthylène,
q est 0,
X est NH,
$R_1$ représente

$$CH-R_3$$
$$|$$
$$CO-R_2$$

où $R_2$ représente un hydroxyle ou un groupement alcoxy inférieur,
$R_3$ représente un radical alkyle linéaire ou ramifié ou phénylalkyle,
ainsi que leurs sels d'addition obtenus avec une base minérale ou organique thérapeutiquement compatible ou avec un acide minéral ou organique thérapeutiquement compatible.

4/ Utilisation, selon l'une des revendications 1 à 3, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) selon les revendications 1 à 3 dans lesquels :
A représente un radical diméthylène,
q est 0, X est NH,
$R_1$ représente

$$CH-R_3$$
$$|$$
$$CO-R_2$$

où $R_2$ représente un hydroxyle ou un groupement alcoxy inférieur,
$R_3$ représente un radical phénylalkyle,
ainsi que leurs sels d'addition obtenus avec une base minérale ou organique thérapeutiquement compatible ou avec un acide minéral ou organique thérapeutiquement compatible.

5/ Utilisation, selon l'une des revendications 1 à 4, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des

troubles de la vision tels que troubles rétiniens d'origine vasculaire, rétinopathies diabétiques, atrophies chroriorétiniennes, dégénérescence choriorétinienne, du composé de formule (I) qui est le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

6/ Utilisation, selon l'une des revendications 1 à 5, pour l'obtention de médicaments destinés au traitement de l'artérite du composé qui est le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

7/ Utilisation, selon l'une des revendications 1 à 6, pour l'obtention de médicaments destinés au traitement de l'artérite des membres inférieurs utilisant le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

8/ Composition pharmaceutique pour le traitement de l'artérite, et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne renfermant un composé selon la revendication 1, ou bien l'un de ses sels d'addition obtenus avec une base minérale ou organique thérapeutiquement compatible ou avec un acide minéral ou organique thérapeutiquement compatible lorsque X représente NH.

9/ Composition pharmaceutique selon la revendication 8 pour le traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, contenant le (S,S,S) [(phényl - 3 éthoxycarbonyl - 1 propyl) amino - 2 propionyl] - 2 aza - 2 carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.

10/ Composition pharmaceutique pour le traitement de l'artérite des membres inférieurs selon l'une des revendications 8 ou 9 contenant le (S,S,S) [(phényl - 3 éthoxycarbonyl - 1 propyl) amino - 2 propionyl] - 2 aza - 2- carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.

**Revendications pour l'Etat contractant suivant: ES**

1/ Utilisation, pour l'obtention de médicaments destinés au traitement de l'artérite et plus particulièrement de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) :

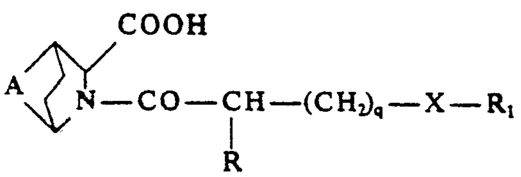

(I)

dans laquelle :
A représente un radical vinylène ou diméthylène,
q est 0 ou 1,
R représente un radical alkyle inférieur pouvant porter un groupe amino,
X représente - S - et $R_1$ représente H, ou bien X représente - NH - et,
$R_1$ représente un atome d'hydrogène ou un radical de formule :

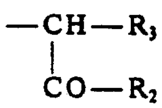

$R_2$ représente un hydroxyle ou un groupement alcoxy inférieur,
$R_3$ représente un atome d'hydrogène, un radical alkyle linéaire ou ramifié, cycloalkyle ou phénylalkyle,

ayant au total un maximum de 8 atomes de carbone, ou un radical de formule :

$$-(CH_2)_p-S-\underset{\displaystyle R_4}{\underset{|}{CH}}-R_5$$

dans lequel :
R4 est H, un radical alkyle inférieur ou cycloalkyle ($C_3$ à $C_6$),
$R_5$ est H, un radical cycloalkyle ($C_3$ à $C_6$) ou alcoxy (inférieur) - carbonyle, et
p est 1 ou 2,
étant entendu, que radical alkyle ou alcoxy inférieur représente des groupes ayant de 1 à 4 atomes de carbone,
ainsi que leurs sels d'addition obtenus avec une base minérale ou organique thérapeutiquement compatible ou avec un acide minéral ou organique thérapeutiquement compatible lorsque X représente NH.

2/ Utilisation, selon la revendication 1, pour l'obtention de médicaments destinés au traitement de l'artérite, de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) dans lequel les carbone dits asymétriques ont la configuration S, ainsi que leurs sels d'addition obtenus avec une base minérale ou organique thérapeutiquement compatible ou avec un acide minéral ou organique thérapeutiquement compatible lorsque X représente NH.

3/ Utilisation, selon les revendications 1 et 2, pour l'obtention de médicaments destinés au traitement de l'artérite et plus particulièrement de l'artérite des membres inférieurs, des troubles de la circulation cérébrale, et particulièrement des troubles de la sénescence cérébrale et la migraine, et des troubles de la vision tels que troubles rétiniens d'origine vasculaire, rétinopathies diabétiques, atrophies choriorétiniennes, dégénérescence choriorétinienne, de composés de formule (1) selon la revendication 1 et 2 dans lesquels :
A représente un radical diméthylène,
q est 0,
X est NH,
$R_1$ représente

$$\begin{array}{l} CH - R_3 \\ | \\ CO - R_2 \end{array}$$

où $R_2$ représente un hydroxyle ou un groupement alcoxy inférieur,
$R_3$ représente un radical alkyle linéaire ou ramifié ou phénylalkyle,
ainsi que leurs sels d'addition obtenus avec une base minérale ou organique thérapeutiquement compatible ou avec un acide minéral ou organique thérapeutiquement compatible.

4/ Utilisation, selon l'une des revendications 1 à 3, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) selon les revendications 1 à 3 dans lesquels :
A représente un radical diméthylène,
q est 0, X est NH,
$R_1$ représente

$$\begin{array}{l} CH - R_3 \\ | \\ CO - R_2 \end{array}$$

où $R_2$ représente un hydroxyle ou un groupement alcoxy inférieur,
$R_3$ représente un radical phénylalkyle,
ainsi que leurs sels d'addition obtenus avec une base minérale ou organique thérapeutiquement compatible ou avec un acide minéral ou organique thérapeutiquement compatible.

5/ Utilisation, selon l'une ces revendications 1 à 4, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies chroriorétiniennes, dégénérescence choriorétinienne, du composé de formule (I) qui est le (S,S,S)

[(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

6/ Utilisation, selon l'une des revendications 1 à 5, pour l'obtention de médicaments destinés au traitement de l'artérite du composé qui est le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

7/ Utilisation, selon l'une des revendications 1 à 6, pour l'obtention de médicaments destinés au traitement de l'artérite des membres inférieurs utilisant le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

8/ Composition pharmaceutique pour le traitement de l'artérite, et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne renfermant un composé selon la revendication 1, ou bien l'un de ses sels d'addition obtenus avec une base minérale ou organique thérapeutiquement compatible ou avec un acide minéral ou organique thérapeutiquement compatible lorsque X représente NH.

9/ Composition pharmaceutique selon la revendication 8 pour le traitement de l'artérite et plus particulièrement de l'artérite des membres inférieurs, des troubles de la circùlation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, contenant le (S,S,S) [(phényl - 3 éthoxycarbonyl - 1 propyl) amino - 2 propionyl] - 2 aza - 2 carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.

10/ Composition pharmaceutique pour le traitement de l'artérite des membres inférieurs selon l'une des revendications 8 ou 9 contenant le (S,S,S) [(phényl - 3 éthoxycarbonyl - 1 propyl) amino - 2 propionyl] - 2 aza - 2 carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.

**Revendications pour l'Etat contractant suivant: GR**

1/ Utilisation, pour l'obtention de médicaments destinés au traitement de l'artérite et plus particulièrement de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) :

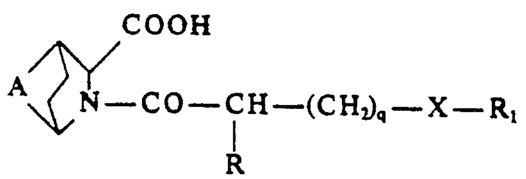

(I)

dans laquelle :
A représente un radical vinylène ou diméthylène,
q est 0 ou 1,
R représente un radical alkyle inférieur pouvant porter un groupe amino,
X représente - S - et $R_1$ représente H, ou bien X représente - NH - et,
$R_1$ représente un atome d'hydrogène ou un radical de formule :

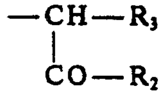

$R_2$ représente un hydroxyle ou un groupement alcoxy inférieur,
$R_3$ représente un atome d'hydrogène, un radical alkyle linéaire ou ramifié, cycloalkyle ou phénylalkyle, ayant au total un maximum de 8 atomes de carbone, ou un radical de formule :

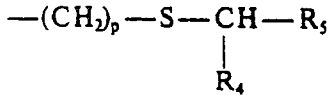

dans lequel :
$R_4$ est H, un radical alkyle inférieur ou cycloalkyle ($C_3$ à $C_6$),
$R_5$ est H, un radical cycloalkyle ($C_3$ à $C_6$) ou alcoxy (inférieur) - carbonyle, et
p est 1 ou 2,
étant entendu, que radical alkyle ou alcoxy inférieur représente des groupes ayant de 1 à 4 atomes de carbone,
ainsi que leurs sels d'addition obtenus avec une base minérale ou organique thérapeutiquement compatible ou avec un acide minéral ou organique thérapeutiquement compatible lorsque X représente NH.

2/ Utilisation, selon la revendication 1, pour l'obtention de médicaments destinés au traitement de l'artérite, de l'artérite des membres inférieurs, des troubles de la circulation cérébrale, et particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) dans lequel les carbone dits asymétriques ont la configuration S, ainsi que leurs sels d'addition obtenus avec une base minérale ou organique thérapeutiquement compatible ou avec un acide minéral ou organique thérapeutiquement compatible lorsque X représente NH.

3/ Utilisation, selon les revendications 1 et 2, pour l'obtention de médicaments destinés au traitement de l'artérite et plus particulièrement de l'artérite des membres inférieurs, des troubles de la circulation cérébrale, et particulièrement des troubles de la sénescence cérébrale et la migraine, et des troubles de la vision tels que troubles rétiniens d'origine vasculaire, rétinopathies diabétiques, atrophies choriorétiniennes, dégénérescence choriorétinienne, de composés de formule (I) selon la revendication 1 et 2 dans lesquels :
A représente un radical diméthylène,
q est 0,
X est NH,
$R_1$ représente

CH - $R_3$
CO - $R_2$

où $R_2$ représente un hydroxyle ou un groupement alcoxy inférieur,
$R_3$ représente un radical alkyle linéaire ou ramifié ou phénylalkyle,
ainsi que leurs sels d'addition obtenus avec une base minérale ou organique thérapeutiquement compatible ou avec un acide minéral ou organique thérapeutiquement compatible.

4/ Utilisation, selon l'une des revendications 1 à 3, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, de composés de formule (I) selon les revendications 1 à 3 dans lesquels :
A représente un radical diméthylène,
q est 0, X est NH,
$R_1$ représente

CH - $R_3$
CO - $R_2$

où $R_2$ représente un hydroxyle ou un groupement alcoxy inférieur,
$R_3$ représente un radical phénylalkyle,
ainsi que leurs sels d'addition obtenus avec une base minérale ou organique thérapeutiquement compatible ou avec un acide minéral ou organique thérapeutiquement compatible.

5/ Utilisation, selon l'une des revendications 1 à 4, pour l'obtention de médicaments destinés au traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies chrorioretiniennes, dégénérescence choriorétinienne, du composé de formule (I) qui est le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement

compatible ou avec un acide minéral ou organique thérapeutiquement compatible.

6/ Utilisation, selon l'une des revendications 1 à 5, pour l'obtention de médicaments destinés au traitement de l'artérite du composé qui est le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

7/ Utilisation, selon l'une des revendications 1 à 6, pour l'obtention de médicaments destinés au traitement de l'artérite des membres inférieurs utilisant le (S,S,S) [(phényl - 3 éthoxycarbonyl - 1 propyl) amino - 2 propionyl] - 2 aza - 2 carboxy - 3 bicyclo [2.2.2] octane, ainsi que ses sels d'addition obtenus avec une base minérale ou organique thérapeutiquement compatible ou avec un acide minéral ou organique thérapeutiquement compatible.

8/ Composition pharmaceutique pour le traitement de l'artérite, et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne renfermant un composé selon la revendication 1, ou bien l'un de ses sels d'addition obtenus avec une base minérale ou organique thérapeutiquement compatible ou avec un acide mineral ou organique thérapeutiquement compatible lorsque X représente NH.

9/ Composition pharmaceutique selon la revendication 8 pour le traitement de l'artérite et plus particulièrement de l'artérite des membres inférieurs, des troubles de la circulation cérébrale, et plus particulièrement des troubles de la sénescence cérébrale et la migraine, des troubles de la vision tels que troubles rétiniens d'origine vasculaire, rétinopathies diabétiques, atrophies choriorétiniennes, dégénérescence choriorétinienne, contenant le (S,S,S) [(phényl - 3 éthoxycarbonyl - 1 propyl) amino - 2 propionyl] - 2 aza - 2 carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.

10/ Composition pharmaceutique pour le traitement de l'artérite des membres inférieurs selon l'une des revendications 8 ou 9 contenant le (S,S,S) [(phényl - 3 éthoxycarbonyl - 1 propyl) amino - 2 propionyl] - 2 aza - 2 carboxy - 3 bicyclo [2.2.2] octane, ou bien l'un de ses sels d'addition obtenus avec une base minérale ou organique thérapeutiquement compatible ou avec un acide minéral ou organique thérapeutiquement compatible.